# NOUVEAU FASCICULE DE BREVET EUROPEEN

(11) **EP 1 926 444 B2**
(45) Date de publication et mention de la décision concernant l'opposition: **24.12.2014**
(45) Mention de la délivrance du brevet: 21.03.2012
(21) Numéro de dépôt: 06831187.7
(22) Date de dépôt: 18.09.2006
(51) Int. Cl.: A61B 17/70

(54) **SYSTEME DE FIXATION VERTEBRALE**
WIRBELFIXATIONSSYSTEM
VERTEBRAL FIXING SYSTEMW

(30) Priorité: 20.09.2005 FR 0509570; 22.02.2006 FR 0650609
(43) Date de publication de la demande: 04.06.2008
(73) Titulaire: ZIMMER SPINE, 33000 Bordeaux (FR)
(72) Inventeur: BACCELLI, Christian, F-33650 Saucats (FR); BELLIARD, Karl, P., F-49770 La Membrolle sur Longuenee (FR); MAZDA, Keyvan, F-75020 Paris (FR)
(74) Mandataire: Mays, Julie
(86) Numéro de dépôt international: PCT/FR2006/050898
(87) Numéro de publication internationale: WO 2007/036657

(56) Documents cités:
- WO-A-2004/010881
- WO-A1-02/09604
- WO-A1-02/24086
- WO-A1-99/53855
- US-B1- 6 514 255
- PATENT ABSTRACTS OF JAPAN vol. 2002, no. 02, 2 avril 2002 (2002-04-02) -& JP 2001 299770 A (AZWELL INC), 30 octobre 2001 (2001-10-30)

## Description

La présente invention concerne un système de fixation vertébrale susceptible d'être monté sur une vertèbre.

Un domaine d'application envisagé est notamment, mais non exclusivement, le traitement des scolioses ou, plus généralement des corrections de courbures anormales du rachis.

Le rachis est formé par la superposition de vertèbres, normalement alignées selon un axe vertébral, des lombaires jusqu'aux cervicales et chacune présentant une paroi postérieure de laquelle fait saillie l'apophyse épineuse et deux bords latéraux des parois desquels font saillies les côtes et/ou les apophyses transverses. Lorsque le rachis d'un individu présente une courbure anormale, les vertèbres sont inclinées les unes par rapport aux autres et par rapport audit axe vertébral. Les bords latéraux des vertèbres situés d'un même côté sont ainsi rapprochés les uns des autres et forment une concavité alors que les bords latéraux de l'autre côté apparaissent éloignés les uns des autres et forment une convexité.

Afin de redresser la colonne vertébrale, les bords latéraux des vertèbres du côté concave sont éloignés les uns des autres et portés les uns par rapport aux autres à une distance sensiblement équivalente à celle qui sépare les bords latéraux de l'autre côté. Pour maintenir ensuite les vertèbres les unes par rapport aux autres, des dispositifs connus comprennent des vis que l'on insère dans les vertèbres ou des crochets que l'on introduit le long de la paroi interne du canal rachidien et des tiges destinées à relier les vis ou les crochets.

Les crochets sont généralement introduits deux par deux dans chaque vertèbre et de chaque côté à proximité des pédicules, leur tête faisant saillie de la paroi postérieure de la vertèbre, une de chaque côté de l'apophyse épineuse. Les têtes formant tulipe, par exemple, sont susceptibles de recevoir une tige qui est bloquée au moyen d'un écrou vissé sur la tête et en appui sur la tige. Les rangées constituées par les têtes de crochet situées de chaque côté des apophyses épineuses sont reliées entre elles et maintenues en position fixe par deux tiges parallèles entre elles et à l'axe du rachis.

Cependant, l'utilisation de ces crochets est délicate puisque l'opérateur ne doit en aucun cas affecter la moelle épinière qui s'étend au centre du canal rachidien, sous peine de provoquer une paralysie du patient.

L'utilisation des vis permet de diminuer les risques de l'intervention. Elles présentent également des têtes formant tulipe et sont insérées, deux par deux sur la paroi postérieure des vertèbres dans les pédicules de chaque côté de l'apophyse épineuse. Ainsi, les vis constituent des points de fixation dans les vertèbres pour les maintenir les unes par rapport aux autres. Cependant, elles sont nécessairement introduites dans le pédicule des vertèbres qui, dans certaines circonstances, est de faible taille ou détérioré.

Un problème qui se pose et que vise à résoudre la présente invention, est alors de constituer des points de fixation, lorsqu'il n'est pas possible d'introduire de vis dans les vertèbres de la portion incurvée et que l'utilisation des crochets s'avère trop dangereuse. Dans la demande de brevet PCT WO2004/010881 au nom de la demanderesse, on a décrit un système de fixation vertébrale qui permet de résoudre ce problème.

Ce système de fixation vertébrale susceptible d'être monté sur une vertèbre du rachis pour la relier à une tige comprend :
- une pièce de liaison disposée au regard de ladite côte et/ou de ladite apophyse transverse, susceptible d'être reliée à ladite tige;
- un lien flexible de forme allongée susceptible de relier ensemble ladite pièce de liaison et au moins une côte et/ou une apophyse transverse ; et
- des moyens de blocage réglables solidaires de ladite pièce de liaison, ledit lien présentant une première extrémité solidaire de ladite pièce de liaison et une seconde extrémité libre susceptible de coulisser dans ladite pièce de liaison en formant une boucle, lesdits moyens de blocage étant susceptibles de maintenir en position fixe, simultanément, ladite pièce de liaison par rapport à ladite tige et une portion dudit lien comprise entre lesdites extrémités étant susceptible d'être bloquée en translation par rapport à ladite pièce de liaison par lesdits moyens de blocage réglables, par quoi la boucle présente une longueur déterminée de façon à bloquer le déplacement relatif de ladite tige et de ladite vertèbre dans des directions opposées l'une de l'autre.

Ce système est satisfaisant mais il peut présenter dans certains cas l'inconvénient suivant. Lorsque le chirurgien exerce une traction sur l'extrémité libre du lien flexible, celui-ci peut être coincé par frottement sur la face inférieure de l'apophyse. Dans ce cas, on comprend que si la portion du lien entre la face inférieure de l'apophyse et la zone de traction sur le lien est effectivement sous tension, la partie qui s'étend entre l'extrémité du lien solidaire de la pièce allongée et la face inférieure de l'apophyse n'est pas sous tension. Il en résulte donc que globalement le lien n'exerce pas convenablement sa fonction de solidarisation avec la vertèbre.

Un objet de la présente invention est de fournir un système de fixation vertébrale qui permet d'éviter les inconvénients cités ci-dessus et d'assurer un blocage contrôlé du lien.

Pour atteindre ce but selon l'invention, le système de fixation vertébrale susceptible d'être monté sur une vertèbre du rachis pour la relier à une tige comprend :
- une pièce de liaison présentant un premier et un deuxième côté, susceptible d'être reliée à ladite tige,
- un lien flexible de forme allongée susceptible de relier ensemble ladite pièce de liaison et au moins une côte et/ou une apophyse transverse et/ou une partie de l'arc postérieur d'une vertèbre ; et
- des moyens de blocage réglables montés sur ladite pièce de liaison, lesdits moyens de blocage étant distincts de ladite pièce de liaison et coopérant avec celle-ci par vissage,
ledit système se caractérisant en ce que :
- ledit lien présente deux extrémités libres ;
- ladite pièce de liaison définit au moins un chemin de passage pour ledit lien de telle manière que deux portions distinctes dudit lien puissent être engagées dans ledit chemin de passage ou lesdits chemins de passage de telle manière que lesdites deux portions de lien définissent une première partie de lien formant une boucle s'étendant d'un premier côté de ladite pièce de liaison et des deuxième et troisième parties de lien s'étendant de l'autre côté de ladite pièce de liaison respectivement entre une desdites portions de lien et une desdites extrémités libres ; et

- lesdits moyens de blocage peuvent prendre une première position par rapport à la pièce de liaison dans laquelle les deux portions de lien sont libres dans ledit ou lesdits chemin(s) de passage, une deuxième position par rapport à la pièce de liaison dans laquelle les deux portions de lien sont immobilisées en translation par rapport à la pièce de liaison et des positions intermédiaires dans lesquelles un coefficient de frottement est créé entre lesdites portions de lien et ladite pièce de liaison, comme défini dans la revendication1.

On comprend que grâce au fait que les deux portions du lien qui se trouvent de part et d'autre de l'apophyse transverse sont toutes les deux disposées dans un ou plusieurs chemins de passage, lorsque les moyens de blocage sont amenés dans leur position de blocage, les deux portions du lien peuvent exercer la tension nécessaire à la fixation de la vertèbre, par l'intermédiaire d'une cote et/ou d'une partie de l'arc postérieur d'une vertèbre et/ou d'une apophyse transverse.

En outre, comme les moyens de blocage coopèrent par vissage avec la pièce de liaison, les "dimensions" du ou des chemins de passage peuvent être définies avec précision dans les différentes phases du serrage puis du blocage du lien.

De préférence, la pièce de liaison définit un unique chemin de passage et les deux portions de lien sont engagées dans le chemin de passage unique.

De préférence également, le chemin de passage unique est défini d'une part par la surface externe de la partie de la tige engagée dans la pièce de liaison et d'autre part par une paroi de la pièce de liaison et les moyens de blocage sont aptes à modifier la section du chemin de passage.

On obtient ainsi, lorsque les moyens de blocage sont dans leur deuxième position, un serrage effectif des deux portions du lien qui entraîne leur immobilisation.

Selon un premier mode de mise en oeuvre, la pièce de liaison comprend deux éléments longitudinaux dont les premières extrémités sont articulées entre elles, les éléments longitudinaux présentant chacun un évidement apte à recevoir une partie d'une section de ladite tige, la paroi dudit évidement définissant avec la surface latérale de la tige, le chemin de passage ou les chemins de passage pour les portions de lien, les moyens de blocage étant montés aux deuxièmes extrémités des éléments longitudinaux.

Selon un deuxième mode de mise en oeuvre, la pièce de liaison comprend une pièce ayant la forme générale d'un U apte à recevoir ladite tige et dont les extrémités externes des branches du U sont filetées et en ce que les moyens de blocage réglables comprennent une bague taraudée apte à coopérer avec le filetage de la pièce en U, le vissage de la bague provoquant le serrage des branches de la pièce sur la tige.

De préférence, le ou lesdits chemins de passage est (sont) constitué(s) par l'espace entre la paroi interne de l'évidement ménagé dans ladite pièce de liaison et la paroi latérale de ladite tige.

D'autres caractéristiques et avantages de l'invention apparaîtront mieux à la lecture de la description qui suit de plusieurs modes de réalisation de l'invention donnés à titre d'exemple non limitatif. La description se réfère aux figures annexées sur lesquelles :
- la figure 1 est une vue en perspective du système de fixation vertébrale selon un premier mode de réalisation ;
- les figures 2A, 2B et 2C sont des vues en coupe verticale du système de fixation illustrant l'utilisation dudit système représenté sur la figure 1 ;
- la figure 3 est une vue de face montrant le système de fixation de la figure 1 mis en place sur une vertèbre ;
- la figure 4 est une vue en perspective d'un deuxième mode de réalisation du système de fixation, le lien n'étant pas représenté ;
- la figure 5 est une vue éclatée du dispositif de liaison de la figure 4 ;
- la figure 6 est une vue de dessus d'une partie du dispositif de liaison de la figure 5 ;
- la figure 6A est une vue en coupe selon la ligne AA de la figure 6 ;
- la figure 7 est une vue de face du système de fixation selon le deuxième mode de réalisation ; et
- les figures 7A et 7B sont des vues en coupe selon la ligne VII-VII de la figure 7 illustrant deux modes de mise en place du lien souple.

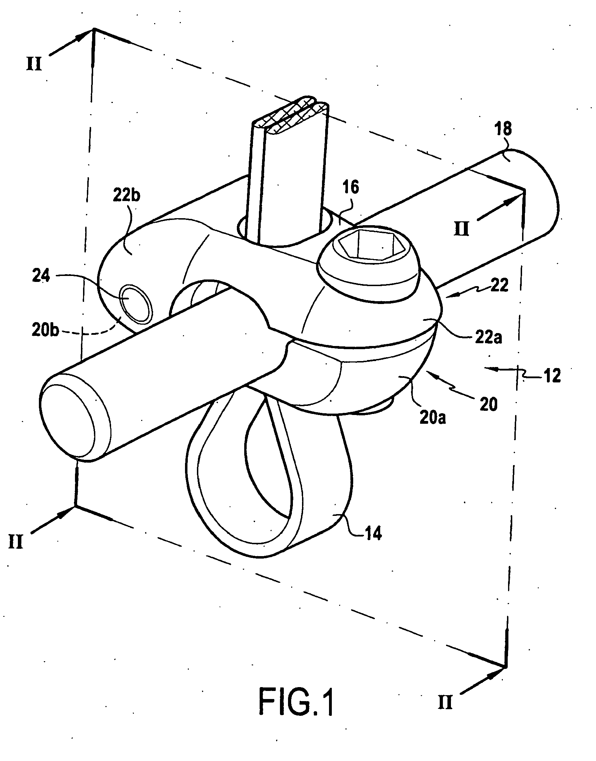

Comme le montre la figure 1, selon le premier mode de réalisation, le système de fixation vertébrale comprend une pièce de liaison 12, un lien flexible 14 et des moyens de blocage réglables 16. Le lien flexible 14 est de forme allongée capable de se conformer au contour des pièces qu'il doit relier. Sur cette figure, on a également représenté la tige 18 qui doit être fixée sur la vertèbre par l'intermédiaire du système de fixation vertébrale. Dans le premier mode de réalisation, la pièce de liaison 12 est constituée par deux éléments longitudinaux respectivement référencés 20 et 22 qui comportent chacun une première extrémité 22a, 20a et une deuxième extrémité 22b, 20b.

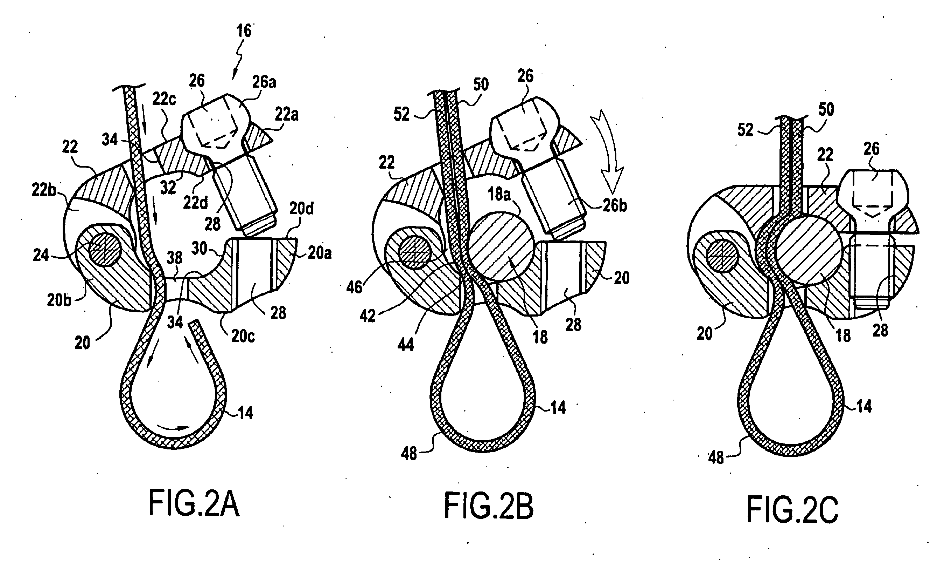

Comme on le voit mieux sur la figure 2A, les éléments longitudinaux 20 et 22 sont articulés l'un par rapport à l'autre à leurs deuxièmes extrémités 20b, 22b autour d'un axe de pivotement 24.

Dans le mode de réalisation considéré, les moyens de blocage sont constitués par une vis 26 dont la tête 26a est engagée dans un trou 28 réalisé à la première extrémité 22a de l'élément longitudinal 22. La première extrémité 20a de l'élément longitudinal 20 est percée d'un alésage taraudé 28 destiné à coopérer avec le corps fileté 26b de la vis 26. Chaque élément longitudinal 20, 22 comporte une face externe 20c, 22c et une face interne 20d, 22d. Les éléments longitudinaux 20 et 22 sont montés de telle manière que les faces internes 20d, 22d des éléments longitudinaux soient en regard l'une de l'autre. Les faces internes 20d, 22d des éléments longitudinaux 20 et 22 comportent des évidements respectivement 30 et 32 en regard l'un de l'autre qui ont une forme sensiblement semi-cylindrique. Les évidements 30 et 32 définissent des parois 34 et 36 qui sont des surfaces réglées dont les génératrices sont parallèles à l'axe de pivotement 24. Enfin, des fentes 38 et 40 font communiquer le fond des évidements 30 et 32 avec les faces externes 20c et 22c des éléments longitudinaux 20 et 22. Comme on l'expliquera plus en détail ultérieurement, les évidements 30 et 32 sont destinés à recevoir la tige 18 ainsi qu'une portion du lien 14, les fentes 38 et 40 étant destinées à laisser passer le lien 14.

En se référant aux figures 2A à 2C, on va expliquer l'utilisation du système de fixation.

Sur la figure 2A, on a représenté les éléments longitudinaux 20 et 22 en position écartée, position dans laquelle bien sûr les moyens de blocage 16 ne sont pas actifs, le corps fileté 26b de la vis 26 n'étant pas engagé dans le trou 28. Le lien 14 est engagé dans les fentes 38 et 40 des éléments longitudinaux contre une partie de la paroi interne 34, 36 des évidements 30 et 32. Puis, on introduit la vis 18 dans l'évidement 30 de l'élément longitudinal 20 de telle manière que les deux portions 42 et 44 du lien 14 soient disposées entre la paroi interne des évidements 30 et 32 et la face latérale 18a de la tige 18. Ces deux surfaces définissent un chemin de passage 46 pour le lien 14 dans lequel sont disposées les portions 42 et 44 du lien 14.

Comme le montre mieux la figure 2B, les portions 42 et 44 du lien définissent pour ce lien 14 une partie formant boucle 48 qui s'étend au-delà de la face externe 20c de l'élément longitudinal 20 et deux parties libres 50 et 52 qui s'étendent hors de la face externe 22c de l'élément longitudinal 22. Lorsque les éléments longitudinaux 20 et 22 sont écartés comme cela est représenté sur la figure 2B, le lien 14 peut coulisser librement dans le chemin de passage 46. Une fois que la portion 48 du lien 14 formant la boucle est disposée autour de l'apophyse transverse ou d'une cote ou encore d'une partie de l'arc postérieur d'une vertèbre, le chirurgien engage le corps fileté 26b de la vis 26 dans l'orifice taraudé 28 provoquant progressivement le rapprochement de l'élément longitudinal 22 vers l'élément longitudinal 20. Ce rapprochement provoque simultanément une diminution de la section du chemin de passage 46 dans lequel sont engagées les portions 42 et 44 du lien et simultanément introduit un certain coefficient de frottement entre le lien et respectivement la tige 18 et les parois des évidements 30 et 32. Néanmoins, il est toujours possible pour le chirurgien d'exercer une traction sur les extrémités libres 50 et 52 du lien 14 jusqu'à ce que l'on obtienne une tension suffisante du lien autour de l'apophyse vertébrale. Lorsque la tension du lien est suffisante pour assurer une fixation convenable, le chirurgien termine le vissage de la vis 26 dans l'orifice taraudé 28 entraînant ainsi le blocage des éléments longitudinaux 20 et 22. Simultanément, on comprend qu'on obtient également le coincement des portions 42 et 44 du lien entre la tige 18 et la paroi des évidements 30 et 32.

Dans cette position de blocage, on obtient donc la solidarisation de la tige 18 et du lien 14 par l'intermédiaire de la pièce de liaison 12.

On comprend également que du fait que le chirurgien exerce une traction sur les deux extrémités libres 50 et 52 du lien 14, on s'affranchit des risques de coincement entre le lien 14 et la face inférieure de l'apophyse transverse ou de la cote, ce qui garantit une solidarisation effective sur l'apophyse transverse ou la cote ou encore une partie de l'arc postérieur d'une vertèbre.

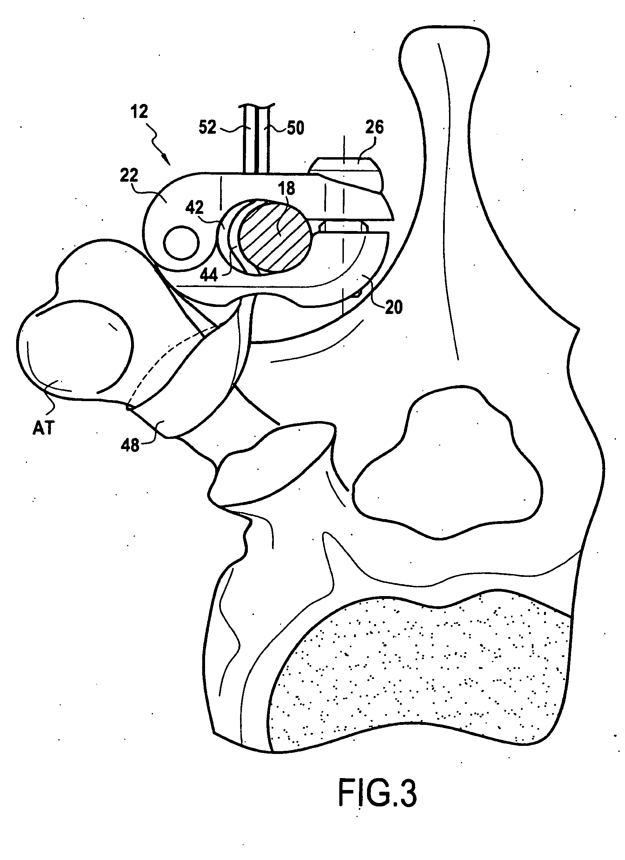

C'est ce qui est représenté sur la figure 3, la référence AT repérant l'apophyse transverse.

Dans la description précédente, les deux portions 42 et 44 du lien sont disposées dans les évidements 30 et 32 d'un même côté de la tige 18. Cette disposition permet d'obtenir un résultat optimal. Cependant, on ne sortirait pas de l'invention si les portions 42 et 44 du lien 14 étaient disposées de part et d'autre de la tige 18. Dans ce cas, il faudrait considérer que la face externe 18a de la tige 18 et les parois internes des évidements 30 et 32 définissent deux chemins de passage respectivement pour les portions 42 et 44 du lien 14.

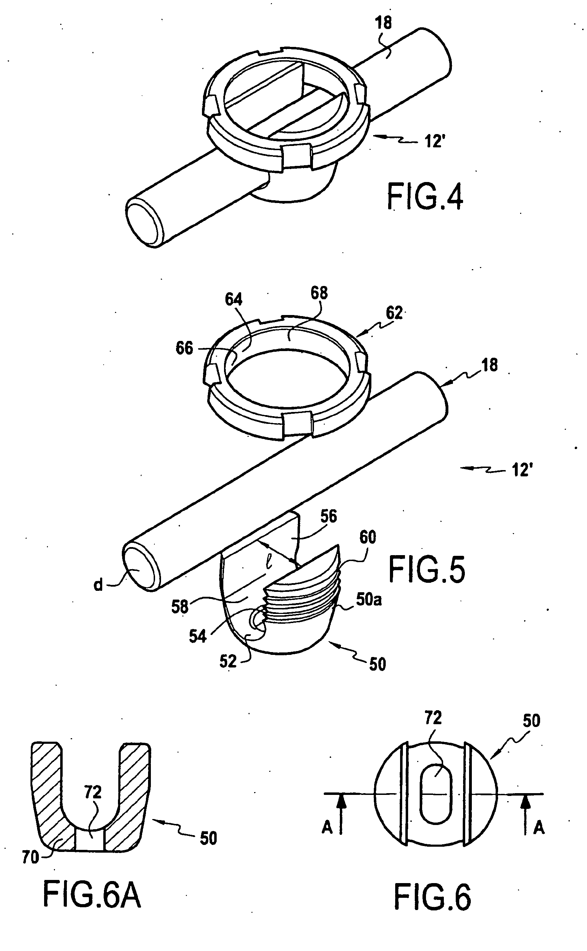

Sur les figures 4 à 7B, on a représenté un deuxième mode de réalisation du système de fixation.

Sur ces figures, on retrouve la tige 18, la pièce de liaison qui porte la référence 12' et le lien souple 14.

Dans ce deuxième mode de réalisation, la pièce de liaison 12' est constituée par une pièce 50 ayant la forme générale d'un U. La paroi interne de cette pièce est constituée par un fond 52 de forme sensiblement semi-cylindrique et par deux portions sensiblement planes 54 et 56 qui correspondent aux deux branches de la pièce 50. La largeur I de l'évidement 58 ménagé dans la pièce 50 est sensiblement égale au diamètre d de la tige 18. Sur sa face externe 50a qui est de révolution autour d'un axe longitudinal de la pièce 50 est réalisé à sa partie supérieure un filetage 60. Le filetage 60 est disposé entièrement au-dessus de la tige 18 lorsque celle-ci est mise en place dans le logement 58. Le filetage 60 est destiné à coopérer avec une bague de serrage 62 qui constitue les moyens de blocage réglables. Cette bague comporte un évidement légèrement tronconique 64 dont la face latérale 66 comporte un taraudage 68.

On comprend dès à présent que la bague 62, lorsqu'elle est entièrement vissée sur la partie filetée 60 de la pièce 50, provoque une déformation élastique des branches de la pièce 50 assurant comme on l'expliquera ultérieurement le pincement et le serrage de portions du lien 14 entre la tige 18 et les ou la paroi interne du logement 58.

Comme le montrent mieux les figures 6 et 6A, la pièce 50 comporte dans son fond 70 un passage 72 pour permettre le passage du lien 14 ainsi qu'on l'expliquera ultérieurement.

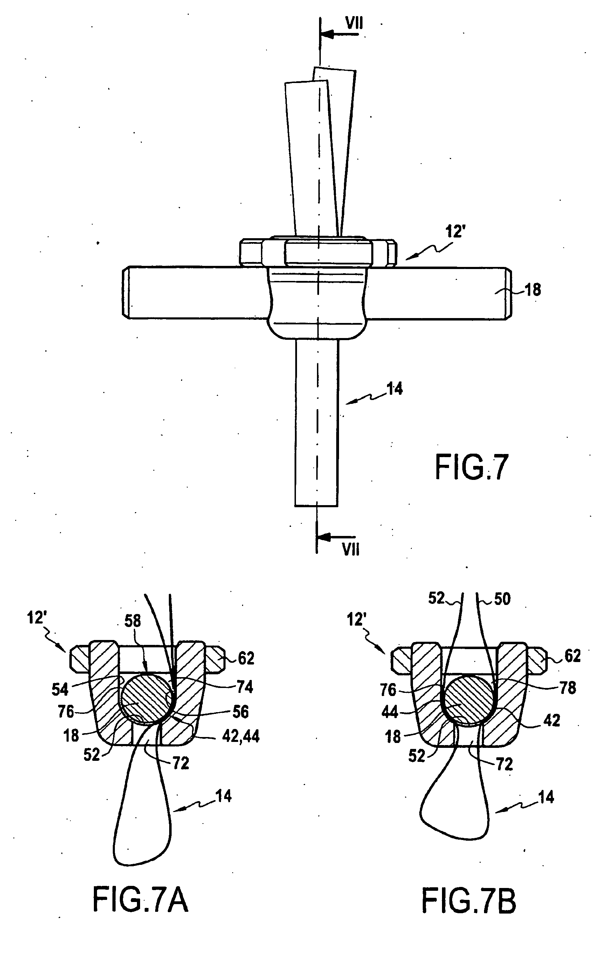

En se référant maintenant aux figures 7, 7A et 7B, on va décrire deux modes différents de mise en place du lien souple 14 à l'intérieur de la pièce de liaison 12' selon le deuxième mode de mise en oeuvre. La paroi latérale de la tige 18 et la paroi interne du logement 58 de la pièce 50 définissent potentiellement deux chemins de passage 74 et 76 pour les parties médianes du lien souple 14. Dans le mode de mise en oeuvre représenté sur la figure 7A, seul le passage 74 est utilisé. Ainsi, les deux portions intermédiaires 42 et 44 du lien souple 14 sont disposées dans le passage 74. Cette disposition présente tous les avantages qui ont été décrits en liaison avec le premier mode de réalisation.

Dans le cas du mode de mise en oeuvre représenté sur la figure 7B, les portions médianes 42 et 44 du lien souple 14 sont disposées respectivement dans les chemins de passage 76 et 78, c'est-à-dire de part et d'autre de la tige 18. Ce mode de réalisation présente également tous les avantages décrits en liaison avec le premier mode de réalisation du dispositif puisque les extrémités libres 50 et 52 du lien 14 sont accessibles pour exercer la traction souhaitée afin d'obtenir un serrage convenable sur l'apophyse épineuse avant de bloquer la bague de serrage 62 sur la pièce 52.

Ce deuxième mode de réalisation présente l'avantage d'être plus simple dans sa conception puisqu'elle permet d'éviter notamment la réalisation des deux pièces longitudinales constituant une sorte de pince articulée autour de l'axe 24.

On comprend que dans les deux modes de réalisation, les moyens de blocage sont constitués par un élément distinct de la pièce de liaison et amovible par rapport à celle-ci. En outre, dans les deux cas, les moyens de blocage coopèrent par vissage avec la pièce de liaison. On peut ainsi régler avec précision les dimensions du ou des chemins de passage définis par la pièce de liaison et la tige dans lequel ou dans lesquels passe le lien. Dans la phase initiale, on peut régler le coefficient de frottement entre le lien d'une part et la tige et la pièce de liaison d'autre part. Dans la phase finale, on obtient un serrage très efficace du lien entre la tige et la pièce de blocage.

## Revendications

1. Système de fixation vertébrale susceptible d'être monté sur une vertèbre du rachis pour la relier à une tige comprenant :
ladite tige;
- une pièce de liaison présentant un premier et un deuxième côté, susceptible d'être reliée à ladite tige ;
- un lien flexible de forme allongée susceptible de relier ensemble ladite pièce de liaison et au moins une côte et/ou une apophyse transverse et/ou une partie de l'arc postérieur d'une vertèbre ; et
- des moyens de blocage réglables montés sur ladite pièce de liaison, lesdits moyens de blocage étant distincts de la pièce de liaison et coopérant avec celle-ci par vissage, ledit système **se caractérisant en ce que** :
- ledit lien présente deux extrémités libres ;
- ladite pièce de liaison définit au moins un chemin de passage pour ledit lien de telle manière que deux portions distinctes dudit lien puissent être engagées dans ledit chemin de passage de telle manière que lesdites deux portions de lien définissent une première partie de lien formant une boucle s'étendant d'un premier côté de ladite pièce de liaison et des deuxième et troisième parties de lien s'étendant de l'autre côté de ladite pièce de liaison respectivement entre une desdites portions de lien et une desdites extrémités libres ;
ledit ou lesdits chemin(s) de passage est (sont) défini(s) d'une part par la surface externe de la partie de la tige engagée dans la pièce de liaison et d'autre part par une paroi de la pièce de liaison et **en ce que** lesdits moyens de blocage sont aptes à modifier la section dudit chemin de passage et
lesdits moyens de blocage peuvent prendre une première position par rapport à la pièce de liaison dans laquelle les deux portions de lien sont libres dans ledit ou lesdits chemin(s) de passage, une deuxième position par rapport à la pièce de liaison dans laquelle les deux portions de lien sont immobilisées en translation par rapport à la pièce de liaison et des positions intermédiaires dans lesquelles un coefficient de frottement est créé entre lesdites portions de lien et ladite pièce de liaison.

2. Système de fixation selon la revendication 1, **caractérisé en ce que** ladite pièce de liaison définit un unique chemin de passage et **en ce que** les deux portions de lien sont engagées dans le chemin de passage unique.

3. Système de fixation selon la revendication 1, **caractérisé en ce que** ladite pièce de liaison comprend deux éléments longitudinaux dont les premières extrémités sont articulées entre elles, lesdits éléments longitudinaux présentant chacun un évidement apte à recevoir une partie d'une section de ladite tige, une paroi dudit évidement définissant avec la surface latérale de ladite tige ledit chemin de passage pour lesdites portions de lien, lesdits moyens de blocage étant montés aux deuxièmes extrémités desdits éléments longitudinaux.

4. Système de fixation selon la revendication 1, **caractérisé en ce que** chaque élément longitudinal comprend une face longitudinale, lesdites faces longitudinales étant en regard l'une de l'autre, et **en ce que** chaque évidement comprend une cavité de forme sensiblement semi-cylindrique débouchant dans ladite face longitudinale et une fente faisant communiquer ladite cavité avec la face opposée de l'élément longitudinal.

5. Système de fixation selon la revendication 1, **caractérisé en ce qu'**il comprend une pièce ayant la forme générale d'un U apte à recevoir ladite tige et dont les extrémités externes des branches du U sont filetées et **en ce que** les moyens de blocage réglables comprennent une bague taraudée apte à coopérer avec le filetage de la pièce en U, le vissage de la bague provoquant le serrage des branches de la pièce sur la tige.

6. Système de fixation selon la revendication 5, **caractérisé en ce que** le ou lesdits chemins de passage est (sont) constitué(s) par l'espace entre la paroi interne de l'évidement ménagé dans ladite pièce de liaison et la paroi latérale de ladite tige.

## Patentansprüche

1. Wirbelbefestigungssystem, das geeignet ist, an einem Wirbel der Wirbelsäule angebracht zu werden, um ihn mit einem Stift zu verbinden, umfassend:
- den Stift;
- ein Verbindungsteil, das eine erste und eine zweite Seite aufweist und dazu geeignet ist, mit dem Stift verbunden zu werden;
- ein flexibles Verbindungsglied langgestreckter Form, das geeignet ist, das Verbindungsteil und wenigstens eine Rippe und/oder einen Querfortsatz und/oder einen Teil des Arcus posterior miteinander zu verbinden; und
- Blockiermittel (36), die an dem Verbindungsteil einstellbar angebracht sind, wobei die Blockiermittel von dem Verbindungsteil verschieden sind und mit diesem durch Verschrauben zusammenwirken, wobei das System **dadurch gekennzeichnet ist, dass**
- das Verbindungsglied zwei freie Enden aufweist;
- das Verbindungsteil wenigstens einen Durchgangsweg für das Verbindungsglied definiert, derart, dass zwei verschiedene Abschnitte des Verbindungsglieds in dem Durchgangsweg in Eingriff kommen können, derart, dass die zwei Verbindungsgliedabschnitte einen ersten Verbindungsteil definieren, der eine Schlaufe bildet, die sich von einer Seite des Verbindungsteils erstreckt, und zweite und dritte Verbindungsteile definieren, die sich von der anderen Seite des Verbindungsteils jeweils zwischen einem der Verbindungsgliedabschnitte und einem der freien Enden erstrecken;
wobei der oder die Durchgangsweg(e) einerseits durch die Außenfläche des Teils der Stange, das in dem Verbindungsstück in Eingriff ist, und andererseits durch eine Wand des Verbindungsteils definiert wird bzw. werden, und dass die Blockiermittel geeignet sind, den Querschnitt des Durchgangswegs zu verändern, und
die Blockiermittel eine erste Position gegenüber dem Verbindungsteil, in der die zwei Verbindungsgliedabschnitte in dem oder den Durchgangsweg(en) frei sind, eine zweite Position gegenüber dem Verbindungsteil, in der die zwei Verbindungsgliedabschnitte gegenüber dem Verbindungsteil gegen ein Verschieben festgelegt sind, und Zwischenpositionen, in denen ein Reibungskoeffizient zwischen den Verbindungsgliedabschnitten und dem Verbindungsteil erzeugt wird, einnehmen können.

2. Befestigungssystem nach Anspruch 1, **dadurch gekennzeichnet, dass** das Verbindungsteil einen einzigen Durchgangsweg definiert und dass die zwei Verbindungsgliedabschnitte in dem einzigen Durchgangsweg in Eingriff sind.

3. Befestigungssystem nach Anspruch 1, **dadurch gekennzeichnet, dass** das Verbindungsteil zwei Längselemente umfasst, von denen die ersten Enden aneinander angelenkt sind, wobei die Längselemente jeweils eine Ausnehmung aufweisen, die für die Aufnahme eines Teils eines Querschnitts des Stifts geeignet ist, wobei eine Wand der Ausnehmung mit der Seitenfläche des Stifts den Durchgangsweg für die Verbindungsgliedabschnitte definiert, wobei die Blockiermittel an den zweiten Enden der Längselemente angebracht sind.

4. Befestigungssystem nach Anspruch 1, **dadurch gekennzeichnet, dass** jedes Längselement eine Längsseite umfasst, wobei die Längsseiten einander gegenüberliegen, und dass jede Ausnehmung einen Hohlraum halbzylindrischer Form, der in der Längsseite ausmündet, und einen Schlitz für die Kommunikation des Hohlraums mit der gegenüberliegenden Seite des Längselements umfasst.

5. Befestigungssystem nach Anspruch 1, **dadurch gekennzeichnet, dass** es ein Teil mit der allgemeinen Form eines U aufweist, das für die Aufnahme des Stifts geeignet ist und von dem die äußeren Enden der Schenkel des U mit Außengewinde versehen sind, und dass die einstellbaren Blockiermittel einen mit Innengewinde versehenen Ring umfassen, der für das Zusammenwirken mit dem Außengewinde des U-förmigen Teils geeignet ist, wobei das Verschrauben des Rings das Umgreifen der Schenkel des Teils an dem Stift bewirken.

6. Befestigungssystem nach Anspruch 5, **dadurch gekennzeichnet, dass** der oder die Durchgangsweg(e) von dem Raum zwischen der Innenwand der Ausnehmung, die in dem Verbindungsteil ausgebildet ist, und der Seitenwand des Stifts gebildet ist bzw. sind.

## Claims

1. A vertebral fixing system suitable for being mounted on a vertebra of the spine in order to connect it to a rod, the system comprising:
a rod;
a connecting part presenting first and second sides and suitable for being connected to said rod;
a flexible ligature of elongate shape suitable for connecting together said connecting part and at least one rib and/or transverse process and/or a portion of the posterior arc of vertebra; and
adjustable locking means mounted on said connecting part, said locking means being distinct from the connecting part and co-operating therewith by screw-fastening; said system being **characterised in that**:
said ligature presents two free ends;
said connecting part defines at least one passageway for passing said ligature in such a manner that two distinct segments of said ligature can be engaged in said passageway(s) so that said two ligature segments define a first ligature portion forming a loop that extends from a first side of said connecting part, and second and third ligature portions extending from the other side of said connecting part between respective ones of said ligature segments and said free ends;
said passageway(s) is(are) defined firstly by the outside surface of the portion of the rod that is engaged in the connecting part and secondly by a wall of the connecting part, and **in that** said locking means are suitable for modifying the section of said passageway and
said locking means are capable of taking a first position relative to the connecting part in which the two ligature segments are free in said passageway(s), a second position relative to the connecting part in which the two ligature segments are prevented from moving in translation relative to the connecting part, and intermediate positions in which a coefficient of friction is created between said ligature segments and said connecting part.

2. A fixing system according to claim 1, **characterized in that** said connecting part defines a single passageway and **in that** both ligature segments are engaged in the single passageway.

3. A fixing system according to claim 1, **characterized in that** said connecting part comprises two longitudinal elements having first ends that are hinged together, each of said longitudinal elements presenting a recess suitable for receiving a portion of a section of said rod, a wall of said recess co-operating with the side surface of said rod to define said passageway for passing said ligature segments, said locking means being mounted at the two second ends of said longitudinal elements.

4. A fixing system according to claim 1, **characterized in that** each longitudinal element has a longitudinal face, said longitudinal faces facing each other, and **in that** each recess comprises a cavity of substantially semicylindrical shape opening out into said longitudinal face and a slot putting said cavity into communication with the opposite face of the longitudinal element.

5. A fixing system according to claim 1, **characterized in that** it comprises a part that is generally U-shaped, suitable for receiving said rod, and having the outer ends of the limbs of the U-shaped threaded, and **in that** the adjustable locking means comprise a tapped ring suitable for co-operating with the thread on the U-shaped part, tightening the ring causing the limbs of the part to be clamped against the rod.

6. A fixing system according to claim 5, **characterized in that** said passageway(s) is (are) constituted by the space between the inside wall of the recess formed in said connecting part and the side wall of said rod.
